# EUROPEAN PATENT APPLICATION

(11) **EP 1 158 053 A1**
(43) Date of publication of application: **28.11.2001**
(21) Application number: 00902874.7
(22) Date of filing: 09.02.2000
(51) Int. Cl.: C12N 15/63

(54) **ACTINOMYCETES-ORIGIN CYCLIC PLASMID DNA**

(30) Priority: 10.02.1999 JP 3272999
(71) Applicant: CHISSO CORPORATION, Osaka-shi, Osaka-fu 530-0005 (JP)
(72) Inventor: INOUYE, Satoshi, Yokohama-shi, Kanagawa 236-0024 (JP); TAKAGI, Hiroshi, Fukui-shi, Fukui 910-0845 (JP); NAKAMORI, Shigeru, Yoshida-gun Fukui 910-1142 (JP)
(74) Representative: Müller-Boré & Partner Patentanwälte
(86) International application number: JP0000708
(87) International publication number: WO0047753

(57) **Abstract**

The invention relates to plasmid pNO33, which is a cyclic plasmid derived from an actinomycete of the genus Streptomyces, having a molecular weight of about 37 Kbp and having restriction enzyme recognition sites shown in Fig. 1.

## Description

### Technical Field

The present invention relates to a plasmid DNA derived from an actinomycete with ε-polylysine generation potency.

### Background of the Invention

Actinomycetes have an ability to generate secondary metabolites, typically including antibiotics and various physiologically active substances. The elucidation of the genetic background is very important for the development of the industrial production. To the modification of bacterial strain, which has been practiced so far, it can confer a theoretical ground to provide more reasonable strategies. Specifically, artificial manipulation of a gene involved in the metabolic pathway of an antibiotic can actively modify the constitution of the final product, while modification of the expression of the gene of an enzyme involved in the production can expectantly improve the productivity.

Generally, actinomycetes are frequently unstable genetically, so actinomycetes can be stored and analyzed by transferring the gene controlling the character into a stable system by recombinant manipulation. Therefore, the development of a plasmid vector essential for the gene recombinant system, using an actinomycete as the host, has been desired. For actinomycetes, however, plasmid vector systems have been developed, mainly for only specific bacterial strains . As to the significant characteristics of actinomycetes, alternatively, the properties prominently vary, depending on the bacterial species, so any standard host-vector system is insufficient for actinomycetes, unlike Escherichia coli currently used frequently. Therefore, the development of a host-vector system suitable for each of the bacterial species as research subject is required.

ε-Polylysine rapidly spreading as a traditional antimicrobial food additive is a lysine polymer derivative generated by specific actinomycetes and is as expensive as 10-fold or more the costs of sorbic acid and glycine known as other common preservatives. So as to elevate the productivity of ε-polylysine, attempts have been made to isolate a bacterial strain with high productivity by traditional mutant treatment. Nevertheless, the production cost of ε-polylysine has not yet been reduced greatly. Hence, it is suggested that the improvement of the productivity by inducing gene recombinant technology is essential. Thus, the development of a vector system usable in bacteria generating ε-polylysine is urgent. Nonetheless, no research or development has been made so far regarding any plasmid vector using an actinomycete generating ε-polylysine as the host.

### Disclosure of the Invention

Therefore, the present inventors have made investigations to isolate a novel plasmid usable for a host-plasmid vector system for an actinomycete bacterial strain with ε-polylysine generating potency and of the genus Streptomyces to thereby enable the application thereof, so as to further improve the productivity of the bacterial strain, which is currently used industrially for the production.

Consequently, the inventors have found a plasmid replicable in the cell of an actinomycete Streptomyces albulus with ε-polylysine generation potency. Thus, the invention has been achieved.

As apparently shown in the aforementioned descriptions, it is a purpose of the invention to provide a plasmid for producing the polylysine, as shown in the following descriptions (1) to (5) and a transformant described below in (6).

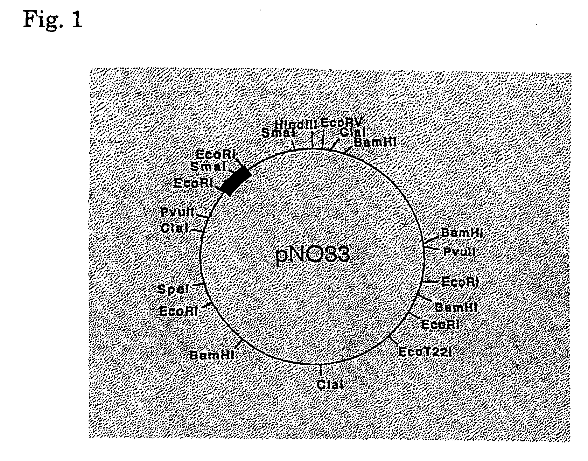

More specifically, the invention has the following constitutions (1) to (6).
(1) Plasmid pN033, which is a cyclic plasmid derived from an actinomycete of the genus Streptomyces, having a molecular size of about 37 Kbp and restriction enzyme recognition sites shown in Fig. 1.
(2) Plasmid pN033 in the first aspect, which is a cyclic piasmid derived from an actinomycete of the genus Streptomyces, having a molecular size of about 37 Kbp and comprising the nucleotide sequence of SQ ID NO. 1.
(3) Plasmid pN033 in the first or second aspect, which is a cyclic plasmid derived from an actinomycete of the genus Streptomyces, having a molecular size of about 37 Kbp and encoding the protein with the amino acid sequence shown by SQ ID NO.2 or 3 or a protein with substantially the same function as that of the protein.
(4) Plasmid pN033 in any one of the first, second and third aspects, where the actinomycete of the genus Streptomyces has ε-polylysine generating potency.
(5) Plasmid pN033 in the fourth aspect, where the actinomycete of the genus Streptomyces and with the ε-polylysine generating potency is Streptomyces albulus IFO 14147.
(6) Plasmid constructed by using the whole nucleotide sequence derived from the cyclic plasmid pN033 or a partial nucleotide fragment thereof.

### Brief Description of the Drawings

Fig. 1 depicts the restriction map of the plasmid pN033 of the invention. The black zone depicts the determinant region of SQ ID NO.1.

### Best Mode for Carrying out the Invention

The invention will now be described in detail hereinbelow. The plasmid pN033 of the invention is a cyclic plasmid recovered from the ε-polylysine-generating bacterium of the genus Streptomyces.

Specifically, the plasmid of the invention can be recovered from Streptomyces albulus IFO 14147 with the ε-polylysine-generating potency. The plasmid is a cyclic plasmid comprising about 37 kbp and restriction enzymes shown in Tables 1 and 2 and Fig. 1.

**Table 1**

| Restriction Enzyme | Cleaved Nucleotide Sequence | Cleavage sites Number | Nucleotide Fragment Size |
|---|---|---|---|
| PstI | CTGCAIG | 0 | 0 |
| StuI | AGGICCT | 0 | 0 |
| ScaI | AGTIACT | 0 | 0 |
| XhoI | CITCGAG | 0 | 0 |
| XbaI | TICTAGA | 0 | 0 |
| HindIII | AIAGCTT | 1 | 37.3 |
| EcoRV | GATIATC | 1 | 37.3 |
| EcoT22I | ATGCAIT | 1 | 37.3 |
| SpeI | AICTAGT | 1 | 37.3 |
| NspV | TTICGAA | 1 | 37.3 |
| PvuII | CAGICTG | 2 | 21.6, 15.7 |
| ClaI | ATICGAT | 3 | 17.4, 11.2, 8.7 |
| BamHI | GIGATCC | 4 | 16.0, 11.4, 6.9, 3.0 |
| EcoRI | GIAATTC | 5 | 14.3, 12.7, 6.6, 1.9, 1.8 |
| A represents adenine; C represents cytosine; G represents guanine; and T represents thymine. | | | |

The determined restriction enzyme positions in Fig. 1 are at clockwise distances from the position of the restriction enzyme HindIII as the starting point, as shown in Table 2.

**Table 2**

| Restriction Enzyme | Distances (kb) |
|---|---|
| HindIII | 0 |
| EcoRV | 0.5 |
| ClaI | 1.0 |
| BamHI | 1.7 |
| BamHI | 8.56 |
| PvuII | 8.8 |
| EcoRI | 10.8 |
| BamHI | 11.6 |
| EcoRI | 12.7 |
| EcoT22I | 14.4 |
| ClaI | 18.4 |
| BamHI | 23.0 |
| EcoRI | 25.4 |
| SpeI | 26.6 |
| ClaI | 29.6 |
| PvuII | 30.4 |
| EcoRI | 32.0 |
| SmaI | 33.1 |
| EcoRI | 33.8 |
| SmaI | 36.6 |

As apparently shown, the plasmid pN033 of the invention carries cleavage sites with various restriction enzymes. The finding indicates that numerous useful vectors can be developed via the modification of the plasmid. Additionally, it is possible to integrate intended genes, for example an enzyme system generating ε-polylysine in the plasmid or a derivative plasmid thereof, and transfecting an appropriate bacterium with the resulting plasmid to transform the host.

Still further, in order to determine whether or not the plasmid recovered in accordance with the invention is novel, it can readily be determined that the plasmid differs from known plasmids derived from the genus Streptomyces, by determining a partial nucleotide sequence of the plasmid pN033 of the invention and comparing the sequence with those of the plasmids . Therefore, the inventors subcloned the DNA fragment of the plasmid pN033 into an appropriate plasmid and determined the nucleotide sequence, as shown as SQ ID NO. 1. Homology screening of the recovered nucleotide sequence (SQ ID NO. 1) with all the nucleotide sequences in the database of the Human Genome Center, the Medical Science Research Institute, University of Tokyo and the database of the National Center for Biotechnology Information (NCBI), USA demonstrated the absence of the same nucleotide sequence. Additionally, absolutely not any plasmid sequence-derived nucleotide sequence with 40 % or more homology to a bacterium-derived nucleotide sequence was detected. In other words, the partial nucleotide sequence of the plasmid pN033 was a nucleotide sequence never having been known so far. Hence, it is shown the plasmid pNO33 of the genus Streptomyces is a novel plasmid DNA.

Furthermore, it was examined whether or not a translatable region as a functional gene was present in the determined SQ ID NO. 1. It was shown that a translatable region was present and the translatable region comprised amino acid sequences of SQ ID NOS. 2 and 3.

SQ ID NO. 2 is a translatable region corresponding to the position 1576 to the position 1803 in SQ ID NO. 1 and is a protein-like polypeptide composed of 76 amino acid residues. SQ ID NO. 3 is a translatable region corresponding to the position 641 to the position 1576 in SQ ID NO. 1 and is a protein-like polypeptide composed of 312 amino acid residues.

This apparently indicates that the plasmid pNO33 can integrate other translatable genes.

In other words, a bacterial strain with high ε-polylysine generation potency can be recovered, by integrating intended useful genes, for example the gene of an enzyme system generating ε-polylysine into the plasmid pNO33 of the invention and transfecting an appropriate host with the resulting plasmid.

Alternatively, homology screening of SQ ID NOS. 2 and 3 with the database of the Genome Center in the same manner indicated that SQ ID NO. 2 was homologous at about 36 % with the gene ORF-D (75 amino acid residues) involved in the regulation of the generation of an antibiotic Actinorhodin, which is present in the chromosome of Streptomyces coelicolor. This indicates that the plasmid pNO33 of the invention may possibly be present as a regulator gene involved in secondary metabolites such as antibiotics. Accordingly, the plasmid pNO33 is industrially applicable.

### Best Mode for Carrying out the Invention

Examples of the invention will now be shown below, but the invention is absolutely never limited to these examples.

### Example 1

### Isolation of plasmid pNO33 from Streptomyces albulus IFO14147

One platinum loop of the gray spore of Streptomyces albulus IFO14147 was inoculated in a 50-ml liquid culture medium [the culture medium was prepared by dissolving bacto-tryptone (10 g) , yeast extract (5 g) and sodium chloride (5 g) in running water (1 liter) and adjusting the resulting solution to pH 7.3] placed in a 500-mi Sakaguchi's flask, for culturing at a shaking velocity of 140 rpm and a temperature of 30 °C for 16 hours.

16 hours later, the liquid culture was centrifuged at 5,000 g for 5 minutes, to separate and harvest the bacteria from the liquid culture. The recovered bacteria were rinsed with an aqueous solution, pH 8.0 containing a TE buffer [Tris(hydroxy)aminoethane (referred to as "Tris" hereinafter)] at a concentration of 25 mM and ethylenediaminetetraacetate disodium (EDTA) at a concentration of 25 mM and were then suspended in 20 ml of a lysis solution (aqueous solution containing sucrose at a concentration of 0.3 M, Tris at a concentration of 25 mM, EDTA at a concentration of 25 mM and lysozyme at a concentration of 2 mg/ml).

The suspension thus recovered was left to stand at 4 °C for 5 minutes. To the resulting suspension was added 10 ml of an aqueous solution containing sodium laurylsulfate at a concentration of 2 % by weight and sodium hydroxide at a concentration of 0.3 N, for mixing, and the resulting solution was left to stand at 4 °C for 5 minutes.

Then, 15 ml of aqueous 5 M ammonium acetate solution was added to the suspension, which was then left to stand at 4 °C for 10 minutes. Then, the suspension was centrifuged at 12,000 g for 10 minutes. After separation, the supernatant was recovered at 35 ml.

To the supernatant was added phenol saturated with an equal volume of TE buffer, and the resulting mixture was mixed together for 5 minutes, by using an agitator (Rotator RT-50, manufactured by Taitech, Co., Ltd.). The mixture solution was centrifuged at 12,000 g for 10 minutes, and the water-soluble fraction was separated at 35 ml (water-soluble fraction 1).

After an equal volume of chloroform was added to the water-soluble fraction 1, the resulting mixture was mixed together with the agitator (Rotator RT-50, manufactured by Taitech, Co., Ltd.) for 5 minutes. The mixture solution was centrifuged at 12,000 g for 10 minutes, and the water-soluble fraction was separated at 35 ml (water-soluble fraction 2).

To the water-soluble fraction 2 were added the 0.1-fold volume of aqueous 3M sodium acetate solution and the 2-fold volume of ethanol, and the resulting water-soluble fraction 2 was left to stand at -20°C for 30 minutes. The deposited white precipitate (nucleic acid fraction) was recovered via centrifugation at 12,000 g for 20 minutes, and the recovered nucleic acid fraction was dissolved in 1 ml of TE buffer, to recover the solution of the nucleic acid fraction in dissolution.

So as to remove ribonucleic acid, ribonuclease (manufactured by Sigma, Co.; trade name of RIBONUCLEASE A) was added to the solution to a final concentration of 50 µg/ml, for reaction at 37 °C for 30 minutes. 30 minutes later, a 0.6-fold volume of an aqueous solution containing polyethylene glycol at a concentration of 20 w/v % and sodium chloride at a concentration of 5 M was added to the resulting reaction mixture, and the resulting solution was left to stand at 4 °C for one hour. During the term, white precipitate was deposited in the mixture solution.

The white precipitate was recovered via centrifugation at 12,000 g for 20 minutes. The recovered white precipitate was dissolved in 1 ml of TE buffer, to recover the plasmid pNO33 of the invention.

The solution of the white precipitate in dissolution was subjected to 0.7 % agarose gel electrophoresis (100 volts, 20 minutes) and subsequent staining of the gel with ethidium bromide. Thus, the presence of high-molecular nucleic acid was verified.

Further, the high-molecular nucleic acid fraction was digested with restriction enzyme HindIII (manufactured by Nippon Gene, Co., Ltd.). The resulting fragments were applied to a pulse-field electrophoresis apparatus manufactured by Bio-Rad, Co., Ltd. (the electrophoresis conditions were according to Bio-Rad, Co., Ltd.; 200 volts and 16 hours), to verify the DNA fragments derived from the plasmid pNO33 of about 37 kbp, using the yeast chromosome as the molecular weight marker.

### Example 2

### Preparation of detailed restriction map of plasmid pNO33

The preparation of detailed restriction map of the plasmid pNO33 recovered in Example 1 comprised singly, doubly or triply digesting pNO33 with various commercially available restriction enzymes (manufactured by Nippon Gene, Co., Ltd. and Takara Shuzo) and subjecting the resulting DNA fragments to 0.7 % agarose gel electrophoresis or pulse-field electrophoresis manufactured by Bio-Rad, Co., Ltd., to determine the molecular weights on the basis of mobility.

The reaction conditions of the enzymes were according to the conditions given by the suppliers. Additionally, the molecular weights were determined on the basis of the standard mobilities of the molecular weight marker (manufactured by Nippon Gene, Co., Ltd.) recovered by digestion of λ phage DNA with restriction enzyme HindIII and the yeast chromosome as a molecular weight marker. Based on these results, the detailed restriction map of the plasmid pNO33 as shown above in Fig. 1 was determined. The results are shown in Fig. 1.

### Example 3

### Determination of the nucleotide sequence (SQ ID NO. 1) and amino acid sequences (SQ ID NOS. 2 and 3) of plasmid pNO33

So as to determine the nucleotide sequence of the plasmid pNO33 as recovered in Example 1, the plasmid pNO33 was digested with restriction enzyme EcoRI. Then, the fragment was subcloned in a known plasmid vector pGreen19 (Inouye et al., Gene, Vol. 189, pp. 159-162: 1997) replicable in Escherichia coli or the EcoRI of pBluescript SK+ (manufactured by Stratagene, Co., Ltd.), according to general methods.

The inserted fragment was confirmed by isolating the plasmid on the basis of the plasmid isolation method described in Example 1, digesting the plasmid with EcoRI and subjecting the digestion products to 0.7 % agarose gel electrophoresis. The nucleotide sequence (SQ ID NO. 1) of the inserted fragment was determined by a fluorescent sequencer (ABI373) manufactured by Applied BioSystems, Co., Ltd., by using the diterminator cycle sequencing method.

Based on the SQ ID NO. 1, translatable regions were screened, by using DNASIS Ver. 3.6 gene analysis software manufactured by Takara Shuzo, so that two amino acid sequences (SQ ID NOS. 2 and 3) were demonstrated.

### Example 4

### Screening of homology of SQ ID NOS. 1, 2 and 3 with gene database

Homology of SQ ID NOS. 1, 2 and 3 with all the worldwide gene databases constructed was screened via Internet on the genome net homepage (http://www.genome.ad.jp/or http://www.ncbi.nlm. nih.gov/), using the analysis software FASTA and BLAST. Screening of homology of SQ ID NO. 1 was carried out with all the entry gene sequences, while screening of homology of SQ ID NOS. 2 and 3 was carried out with the entry amino acid sequences and amino acid sequences translated from the entry gene sequences. Consequently, no gene sequence with significant homology with SQ ID NO.1 was detected.

No prominent homology with SQ ID NOS. 2 and 3 was detected. However, about 36 % homology of SQ ID NO. 2 with the gene ORF-D present in the chromosome of Streptomyces coelicolor and involved in the generation of an antibiotic Actinorhodin was detected. This indicates the presence of gene of secondary metabolites such as antibiotics on the plasmid of the invention, suggesting the industrial applicability.

### Industrial Applicability

The plasmid of the invention has cleavage sites with various restriction enzymes. Via the modification of the plasmid, numerous useful vectors can be developed, which are applicable to bacteria of the genus Streptomyces. Additionally, useful substances can be generated by integrating useful genes into the plasmid and transfecting bacteria of the genus Streptomyces with the resulting plasmid to transform the hosts.

## Claims

1. Plasmid pNO33, which is a cyclic plasmid derived from an actinomycete of the genus Streptomyces, having a molecular size of about 37 Kbp and restriction enzyme recognition sites shown in Fig. 1.

2. Plasmid pN033 according to claim 1, which is a cyclic plasmid derived from an actinomycete of the genus Streptomyces, having a molecular size of about 37 Kbp and comprising the nucleotide sequence of SQ ID NO. 1.

3. Plasmid pNO33 according to claim 1 or 2, which is a cyclic plasmid derived from an actinomycete of the genus Streptomyces, having a molecular size of about 37 Kbp and encoding the protein with the amino acid sequence shown by SQ ID NO.2 or 3 or a protein with substantially the same function as that of the protein.

4. Plasmid pNO33 according to any of claims 1 to 3, where the actinomycete of the genus Streptomyces has ε-polylysine generating potency.

5. Plasmid pNO33 according to claim 4, where the actinomycete of the genus Streptomyces and with the ε-polylysine generating potency is Streptomyces albulus IFO 14147.

6. Plasmid constructed by using the whole nucleotide sequence derived from the cyclic plasmid pNO33 or a partial nucleotide fragment thereof.


SEQUENCE LISTING

<110> Chisso Corporation

<120> Cyclic plasmid DNA originated from Streptomyces

<130> CS-4400

<140>
<141>

<150> JP P1999-032729
<151> 1999-02-10

<160> 3

<170> PatentIn Ver. 2.1

<210> 1
<211> 1806
<212> DNA
<213> Streptomyces albulus

<400> 1
gaattcgatg gtggcccggg gcggacacca caggatcggc cccagcgtca cgccgttcgt 60
ggtcagggcc tcgatcgcgt cccaaccaac ggtggcggtc acccgcaaca cgtcccagct 120
ccgcccgatc ggcacgtcaa cggcgtgccc ggcggcccat gcagcgtgta cgtcatgctt 180
gtccgctcct gcggctgttg cccatcggag cgcgcggacg aagtgctgtc cgatcgtcat 240
cgcgcgccct cctcgtgctc cgggcacgcc cgcacgtccc aactgatgcc ggcgcccgat 300
gcggagacgg tgcgcacgcg cccggcaggg cacaactggg tgcgctggtt gcgggtccgc 360
ccgcagacga tgcaggcgtg cccctgaagt tgccctaacg cggggaaatc agggtctccg 420
acccgatatg cgcgcttgat cggcttttgc tgtgtggtca gtgccattgc tgcactccgc 480
tcactagccc aggtgctccc acgatcagca acggaaccgc tacgtcgcgt atcgggatta 540
cccctagagt gacccctcac acacgtgccg cgcaagtgtc acgcacgtgc gtagcacgtg 600
cgtgacgttc gtacaggtgg acggagaggc ggagaaccct atgtgtgcag ctcgtcgagg 660
tgatacggca gactggaccg aagcaagtgc gcgcaaacga cgcagacgga gggggcgggg 720
tatgccaccg gagccgacca gcccgacggt tcaccgtcgc caactgggcc gcgaactgcg 780
ccaactgagg caggcggcgg ggaaagagat cgaggatgcg gcccagacgt tgggctgtca 840
ccgaagccgg gttagccgga tcgagaacgg gcggggcggc gcggtcgcaa agccgcaaga 900
tgtaaaagac ctgctcacgc tctacggcgt ggacgacgcc ataaagctcg cccgactcct 960
ggcactgctg aacgactccc agaagcccgg atggtgggag agcctggagt ttccgccggg 1020
actcgatgcg ctcgtgggcc tggagaccga tgcgcgcaag gaatcggctt ttgagccgtt 1080
gctgatcccc gggccgctac aggttcctga ctacgcgcgg gctgtgatcc atgcagctcg 1140
ctctcagcat ccggacgagg tcgaagacct ggttcagctc cgggacgggc gcctacgcct 1200
gctgacacgt tctcaaaacc cgttggagct gtgggccgta gtagatgagt ccgtgatccg 1260
tcggcccgtc ggcggcgctc ggacactccg tgatcaactg cggcatctga tggaaatcgc 1320
ggcattgccg aacgtgacgc ttcaggtcgt tccttttgag aaagagagtc accccggact 1380
cggcggctcc tttgcactct tggagttcga ggatgatccg gcggtggtct atgtggacag 1440
catggccggg aacctgtacc tggaaaaagc acgcgacgtg aggcgcttcg tgcgggcctt 1500
cgatctgctg aaggcgtgcg cgctcgaccc tgacgactca gcggccttga tcaaccgtgc 1560
cgcaaaggag agcaaatgag taacaccacc ccgacgccga acgccctgct ggctggcgcc 1620
cagtggttca agtccgaggc gtcaagcgca agcgggggtt gcctcgaagt ggcgtttcta 1680
cccggcgatc tcgtcggcat ccgagacaac gaggacctga ccaacccgcc ctttgtcgtc 1740
accaagcatg tgtggaactg ctgggtcgcc ggcgccaagg caggagaatt cgacccgccc 1800
gcctga 1806

<210> 2
<211> 76
<212> PRT
<213> Streptomyces albulus

<400> 2
Met Ser Asn Thr Thr Pro Thr Pro Asn Ala Leu Leu Ala Gly Ala Gln
1               5                   10                  15

Trp Phe Lys Ser Glu Ala Ser Ser Ala Ser Gly Gly Cys Leu Glu Val
            20                  25                  30

Ala Phe Leu Pro Gly Asp Leu Val Gly Ile Arg Asp Asn Glu Asp Leu
        35                  40                  45

Thr Asn Pro Pro Phe Val Val Thr Lys His Val Trp Asn Cys Trp Val
    50                  55                  60

Ala Gly Ala Lys Ala Gly Glu Phe Asp Pro Pro Ala
65                  70                  75


<210> 3
<211> 312
<212> PRT
<213> Streptomyces albulus

<400> 3
Met Cys Ala Ala Arg Arg Gly Asp Thr Ala Asp Trp Thr Glu Ala Ser
1               5                   10                  15

Ala Arg Lys Arg Arg Arg Arg Arg Gly Ala Gly Met Pro Pro Glu Pro
            20                  25                  30

Thr Ser Pro Thr Val His Arg Arg Gln Leu Gly Arg Glu Leu Arg Gln
        35                  40                  45

Leu Arg Gln Ala Ala Gly Lys Glu Ile Glu Asp Ala Ala Gln Thr Leu
    50                  55                  60

Gly Cys His Arg Ser Arg Val Ser Arg Ile Glu Asn Gly Arg Gly Gly
65                  70                  75                  80

Ala Val Ala Lys Pro Gln Asp Val Lys Asp Leu Leu Thr Leu Tyr Gly
                85                  90                  95

Val Asp Asp Ala Ile Lys Leu Ala Arg Leu Leu Ala Leu Leu Asn Asp
            100                 105                 110

Ser Gln Lys Pro Gly Trp Trp Glu Ser Leu Glu Phe Pro Pro Gly Leu
        115                 120                 125

Asp Ala Leu Val Gly Leu Glu Thr Asp Ala Arg Lys Glu Ser Ala Phe
    130                 135                 140

Glu Pro Leu Leu Ile Pro Gly Pro Leu Gln Val Pro Asp Tyr Ala Arg
145                 150                 155                 160

Ala Val Ile His Ala Ala Arg Ser Gln His Pro Asp Glu Val Glu Asp
                165                 170                 175

Leu Val Gln Leu Arg Asp Gly Arg Leu Arg Leu Leu Thr Arg Ser Gln
            180                 185                 190

Asn Pro Leu Glu Leu Trp Ala Val Val Asp Glu Ser Val Ile Arg Arg
        195                 200                 205

Pro Val Gly Gly Ala Arg Thr Leu Arg Asp Gln Leu Arg His Leu Met
    210                 215                 220

Glu Ile Ala Ala Leu Pro Asn Val Thr Leu Gln Val Val Pro Phe Glu
225                 230                 235                 240

Lys Glu Ser His Pro Gly Leu Gly Gly Ser Phe Ala Leu Leu Glu Phe
                245                 250                 255

Glu Asp Asp Pro Ala Val Val Tyr Val Asp Ser Met Ala Gly Asn Leu
            260                 265                 270

Tyr Leu Glu Lys Ala Arg Asp Val Arg Arg Phe Val Arg Ala Phe Asp
        275                 280                 285

Leu Leu Lys Ala Cys Ala Leu Asp Pro Asp Asp Ser Ala Ala Leu Ile
    290                 295                 300

Asn Arg Ala Ala Lys Glu Ser Lys
305                 310